(19) Europäisches Patentamt
European Patent Office
Office européen des brevets

(11) **EP 2 676 685 A1**

(12) **EUROPÄISCHE PATENTANMELDUNG**

(43) Veröffentlichungstag:
**25.12.2013  Patentblatt 2013/52**

(51) Int Cl.:
*A61L 31/02* (2006.01)   *C22C 38/00* (2006.01)
*C22C 38/18* (2006.01)   *C22C 38/22* (2006.01)
*C22C 38/34* (2006.01)   *C22C 38/38* (2006.01)
*C22C 38/44* (2006.01)   *C22C 38/58* (2006.01)

(21) Anmeldenummer: **13165600.1**

(22) Anmeldetag: **26.04.2013**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **18.06.2012   US 201261660818 P**

(71) Anmelder: **Biotronik AG
8180 Bülach (CH)**

(72) Erfinder:
• **Gerold, Bodo**
**97753 Karlstadt (DE)**
• **Müller, Heinz**
**91052 Erlangen (DE)**
• **Uggowitzer, Peter**
**8913 Ottenbach (CH)**

(74) Vertreter: **Lindner-Vogt, Karin L.
Biotronik SE & Co. KG
Corporate Intellectual Properties
Woermannkehre 1
12359 Berlin (DE)**

(54)   **Stent aus einer Eisenlegierung**

(57)   Die Erfindung betrifft einen Stent, der ganz oder in Teilen aus einer Eisenlegierung der folgenden Zusammensetzung besteht (in Gew.%): Cr: > 12.0; Ni: 0 - 8.0; Co: 0 - 20.0; Mn: 0 - 20.0; N: 0.05 - 1.0; C 0.05 - 0.4; Ti: 0 - 3.5; Nb: 0 - 3.5; V: 0 - 3.5; Mo 0 - 3.5; Si: 0-3.0; Al: 0-3.0; und Cu:0-3.0.

EP 2 676 685 A1

**Beschreibung**

Die Erfindung betrifft einen Stent, der ganz oder in Teilen aus einer Eisenlegierung besteht.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneuryrismas dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Geweboveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare (hier als biokorrodierbar bezeichnete) Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit bioinerten Implantatwerkstoffen, insbesondere Basislegierungen des Eisens.

Stents müssen die Fähigkeit besitzen große plastische Dehnungen zu ertragen und ihren Größe bzw. Durchmesser zu behalten, wenn sie aufgeweitet sind. Grundsätzlich gilt, der ideale Stent sollte:

○ ein geringes Profil besitzen; Dies umschließt die Tauglichkeit auf einen Ballonkatheter gecrimped zu werden.
○ ein gutes Aufweitverhalten zeigen; Wenn der Stent in die Läsion eingeführt und der Ballon aufgeblasen wird, soll sich der Stent gleichmäßig aufweiten, um sich der Gefäßwand anzupassen.
○ eine ausreichende Radialfestigkeit und einen vernachlässigbaren Recoil besitzen; Wenn der Stent einmal gesetzt ist, soll er den Rückstellkräften der Gefäßwand widerstehen und nicht kollabieren.
○ eine ausreichende Flexibilität besitzen; So kann der Stent auch durch Gefäße und Stenosen mit geringen Durchmesser befördert werden.
○ eine angemessene Röntgensichtbarkeit bzw. MRI-Kompatibilität besitzen; So kann der Mediziner die Implantation und Lage des Stents in vivo beurteilen.
○ geringe Thrombogenität besitzen; Das Material sollte biokompatibel sein und insbesondere die Anlagerung und Verklumpung von Blutplättchen vermeiden.
○ die Möglichkeit zur Wirkstofffreisetzung haben; Dies dient insbesondere der Restenosevermeidung.

Die Anforderungen sprechen insbesondere die mechanischen Eigenschaften des Materials an, aus dem der Stent gefertigt ist. Günstig ist es eine hohe Streckgrenze (Last bei der die plastische Verformung des Werkstoffes beginnt) und gleichzeitig eine hohe maximale Festigkeit zu haben. Das Verhältnis Streckgrenze / maximaler Festigkeit (Streckgrenzenverhältnis) sollte möglichst klein sein, da im anderen Fall ein immer größerer Anteil einer Verformung elastisch erfolgt und damit ein hoher elastischer Recoil resultiert.

Die klassischen Materialien 316L (Fe-Basis-Legierung), MP35N und L-605 (Co-BasisLegierungen), die für den Bau ballonexpandierbarer Stents verwendet werden, besitzen zwar bereits eine hohe Festigkeit und eine hohe Bruchdehnung, zeigen aber gerade bei der Optimierung der oben genannten Eigenschaften (gleichzeitige Verbesserung von Festigkeit, Streckgrenze und Streckgrenzenverhältnis) Grenzen. Dies engt die Freiheit bei der Stent-Design-Entwicklung und im Einsatz ein:

(i) zu geringe (Zug-)Festigkeit Rm (UTS) und plastische Dehnung At (elongation at fracture)

In der Folge sind der Kollapsdruck bzw. die Radialfestigkeit geringer, so dass dickere Stentstreben notwendig sind, um einen Lumenverlust oder einen Stentkollaps durch die Rückdehnungskräfte des aufgeweiteten Gefäßes zu vermeiden. Dadurch ist das Crimpprofil dicker, so dass es zu einem größeren Lumenverlust kommt, was die Einheilung (Endothelialisierung) in die Gefäßwand verzögert.

(ii) eine Steigerung der (Zug-)Festigkeit lässt sich nur erreichen, wenn gleichzeitig eine geringere Bruchdehnung oder eine überproportionale Anhebung der Streckgrenze Rp0.2 (YTS) in Kauf genommen werden. Dies führt aber zu einer höheren Bruchneigung des Stents oder zu einem starken Rückfedern nach der Expansion. Eine starke elastische Rückfederung führt neben einem Lumenverlust nach Implantation auch zu einer schlechteren Crimpbarkeit und erhöht somit die Gefahr von Stentverlusten vom Katheter.

Demnach besteht anhaltender Bedarf an einem metallischen Implantatwerkstoff, der zur Herstellung von Stents geeignet ist.

Der erfindungsgemäße Stent löst oder mindert ein oder mehrere der zuvor geschilderten Aufgaben. Der Stent besteht ganz oder in Teilen aus einer Eisenlegierung der Zusammensetzung:

Cr: > 12.0 Gew.%
Ni: 0 - 8.0 Gew.%
Co: 0 - 20.0 Gew.%
Mn: 0 - 20.0 Gew.%
N: 0.05 - 1.0 Gew.%
C: 0.05 - 0.4 Gew.%
Ti: 0 - 3.5 Gew.%
Nb: 0 - 3.5 Gew.%
V: 0 - 3.5 Gew.%
Mo: 0 - 3.5 Gew.%
Si: 0 - 3.0 Gew.%
Al: 0 - 3.0 Gew.%
Cu: 0 - 3.0 Gew.%

wobei ein kumulierter Gehalt von Co und Mn bei 3.0 - 20.0 Gew.% liegt sowie Eisen und herstellungsbedingte Verunreinigungen den auf 100 Gew.% verbleiben Rest einnehmen und

(i) eine Wertzahl Cr-eq für Cr-Äquivalente, die sich aus den Gew.%-Anteilen der genannten Legierungskomponenten nach der Formel (1)

$$Cr\text{-}eq = [Cr] + 1.5 \times [Mo] + 0.48 \times [Si] + 2.5 \times [Al] + 1.75 \times [Nb] + 2.3 \times [V] \qquad (1)$$

ergibt, größer als 18 ist;

(ii) eine Wertzahl Ni-eq für Ni-Äquivalente, die sich aus den Gew.%-Anteilen der genannten Legierungskomponenten nach der Formel (2)

$$Ni\text{-}eq = [Ni] + [Co] + 30 \times [C] + 18 \times [N] + 0.1 \times [Mn] - 0.01 \times [Mn]^2 \qquad (2)$$

ergibt, kleiner als 22 ist;

(iii) eine Wertzahl PRE für Korrosionsbeständigkeit, die sich aus den Gew.%-Anteilen der genannten Legierungskomponenten nach der Formel (3)

$$PRE = [Cr] + 3.3 \text{ x } [Mo] + 20 \text{ x } [N] \quad (3)$$

ergibt, größer als 25 ist;

(iv) für die Wertzahlen Cr-eq und Ni-eq die Beschränkung nach der Formel (4) gilt

$$Ni\text{-}eq > Cr\text{-}eq - 8 \qquad\qquad (4)$$

und

(v) für den Gehalt an Stickstoff und Kohlenstoff folgende Einschränkungen der Formeln (5) und (6) gelten

$$0.25 \leq C + N \leq 1.00 \qquad\qquad (5)$$

$$0.25 \leq C/N \leq 1.00 \qquad\qquad (6)$$

Die erfindungsgemäß verwendete Fe-Basislegierungen ist korrosionsbeständig, reibverschleißbeständig und besitzt ein hohes Kaltumformvermögen, ausgezeichnete Zähigkeitseigenschaften und eine hohe Festigkeit. Ein Anteil von Austenit an der Legierung liegt vorzugsweise bei mehr als 95%, insbesondere liegt die Legierung vollständig in austenitischer Modifikation vor, da derartige Legierungen für Magnetresonanzuntersuchungen geeignet sind. Der erfindungsgemäßen CrMnNi-Stahl zeigen TRIP-Effekte (Transformation Induced Plasticty) - und TWIP-Effekte (Twinning Induced Plasticity). Den austenitischen Zustand stabilisieren die Legierungskomponenten Co, Mn und N. Als die Stapelfehlerenergie erhöhende Legierungskomponenten werden Si, Al und Cu zugesetzt.

Die erfindungsgemäß verwendeten Legierungen besitzen eine sehr hohe Festigkeit Rm von > 800 MPa, bevorzugt > 900 MPa. Die hohe Festigkeit ermöglicht es im Stent-Design dünne Strukturen zu realisieren, die dem Stent dennoch eine hohe Radialfestigkeit von > 1.5 bar (150 kPa) verleihen.

Die erfindungsgemäßen Legierungen zeigen weiterhin eine ausgezeichnete Umformbarkeit bei Raumtemperatur. Der Verformungsgrad (Bruchdehnung) A liegt bei > 40%, bevorzugt > 60%.

Die erfindungsgemäßen Legierungen besitzen einen hohen Widerstand gegen lokale Korrosion, den sogenannten Lochfraß. Dieser Widerstand lässt sich durch Vorgabe der genannten Wertzahl PRE (Pitting Resistance Equivalent) vorgeben. PRE ist bevorzugt größer als 28, insbesondere 30.

Cr-eq ist größer als 18, vorzugsweise größer als 20, und Ni-eq ist kleiner als 22, vorzugsweise kleiner als 18. Durch die Ungleichung der Formel (4) ist sichergestellt, dass  immer im austenitischen Gebiet gearbeitet wird, also kein Ferrit vorliegt und damit Ferromagnetismus vermieden werden kann. Will man ein hohes PRE, sind Cr-eq und Ni-eq ebenfalls hoch.

Die Legierungen sind analog den üblichen Herstellverfahren für Eisenbasis-Legierungen herstellbar.

Ausführungsbeispiel 1

Eine Ni-freie Legierung mit der Zusammensetzung (in Gew.%) 17%Cr, 0.5%Mo, 10%Mn, 2%Si, 0.25%C und 0.4%N wurde im Vakuumschmelzofen unter Stickstoffatmosphäre mit einem Partialdruck von ca. 1 bar erschmolzen und zu Barren der Dimension 8cm x 8cm abgegossen. Nach einer Schmiedeumformung zu Stangen der Dimension 2.5cm x 2.5cm wurden diese bei einer Temperatur von 1.150°C für 6h lösungsgeglüht und in Wasser abgeschreckt. Das Material zeigt ein homogenes Gefüge mit einer Korngröße von ca. 20 μm. Für die solcherart hergestellte Legierung gelten die folgenden Kennwerte:

Cr-eq: 18.7; Ni-eq: 16.5; PRE: 26.7
Rp0.2 = 540 MPa; Rm = 920 MPa; A = 65%

Ausführungsbeispiel 2

Eine Legierung mit der Zusammensetzung (in Gew.%) 17%Cr, 1.5Mo, 5.5%Ni. 7%Mn, 2%Si, 0.1%C und 0.25%N wurde im Vakuumschmelzofen unter Stickstoffatmosphäre mit einem Partialdruck von ca. 1 bar erschmolzen und zu Barren der Dimension 8cm x 8cm abgegossen. Nach einer Schmiedeumformung zu Stangen der Dimension 2.5cm x 2.5cm wurden diese bei einer Temperatur von 1.150°C für 6h lösungsgeglüht und in Wasser abgeschreckt. Das Material zeigt ein homogenes Gefüge mit einer Korngröße von ca. 25 $\mu$m. Für die solcherart hergestellte Legierung gelten die folgenden Kennwerte:

Cr-eq: 20.2; Ni-eq: 13.2; PRE: 26.0
Rp0.2 = 405 MPa; Rm = 890 MPa; A = 70%

**Patentansprüche**

1. Stent bestehend ganz oder in Teilen aus einer Eisenlegierung der Zusammensetzung
   Cr: > 12.0 Gew.%
   Ni: 0 - 8.0 Gew.%
   Co: 0 - 20.0 Gew.%
   Mn: 0 - 20.0 Gew.%
   N: 0.05 - 1.0 Gew.%
   C: 0.05 - 0.4 Gew.%
   Ti: 0 - 3.5 Gew.%
   Nb: 0 - 3.5 Gew.%
   V: 0 - 3.5 Gew.%
   Mo: 0 - 3.5 Gew.%
   Si: 0 - 3.0 Gew.%
   Al: 0 - 3.0 Gew.%
   Cu: 0 - 3.0 Gew.%
   wobei ein kumulierter Gehalt von Co und Mn bei 3.0 - 20.0 Gew.% liegt sowie Eisen und herstellungsbedingte Verunreinigungen den auf 100 Gew.% verbleiben Rest einnehmen und

   (i) eine Wertzahl Cr-eq für Cr-Äquivalente, die sich aus den Gew.%-Anteilen der genannten Legierungskomponenten nach der Formel (1)

   $$Cr\text{-}eq = [Cr] + 1.5 \times [Mo] + 0.48 \times [Si] + 2.5 \times [Al] + 1.75 \times [Nb] + 2.3 \times [V] \qquad (1)$$

   ergibt, größer als 18 ist;
   (ii) eine Wertzahl Ni-eq für Ni-Äquivalente, die sich aus den Gew.%-Anteilen der genannten Legierungskomponenten nach der Formel (2)

   $$Ni\text{-}eq = [Ni] + [Co] + 30 \times [C] + 18 \times [N] + 0.1 \times [Mn] - 0.01 \times [Mn]^2 \qquad (2)$$

   ergibt, kleiner als 22 ist;
   (iii) eine Wertzahl PRE für Korrosionsbeständigkeit, die sich aus den Gew.%-Anteilen der genannten Legierungskomponenten nach der Formel (3)

   $$PRE = [Cr] + 3.3 \times [Mo] + 20 \times [N] \qquad (3)$$

   ergibt, größer als 25 ist;
   (iv) für die Wertzahlen Cr-eq und Ni-eq die Beschränkung nach der Formel (4) gilt

$$\text{Ni-eq} > \text{Cr-eq} - 8 \qquad\qquad (4)$$

und
(v) für den Gehalt an Stickstoff und Kohlenstoff folgende Einschränkungen der Formeln (5) und (6) gelten

$$0.25 \leq C + N \leq 1.00 \qquad\qquad (5)$$

$$0.25 \leq C/N \leq 1.00 \qquad\qquad (6).$$

2. Stent nach Anspruch 1, bei dem die Wertzahl Cr-eq größer als 20 ist.

3. Stent nach einem der vorhergehenden Ansprüche, bei dem die Wertzahl Ni-eq kleiner als 18 ist.

4. Stent nach einem der vorhergehenden Ansprüche, bei dem die Wertzahl PRE größer als 28 ist.

5. Verwendung einer Eisenlegierung der Zusammensetzung
   Cr: > 12.0 Gew.%
   Ni: 0 - 8.0 Gew.%
   Co: 0 - 20.0 Gew.%
   Mn: 0 - 20.0 Gew.%
   N: 0.05 - 1.0 Gew.%
   C: 0.05 - 0.4 Gew.%
   Ti: 0 - 3.5 Gew.%
   Nb: 0 - 3.5 Gew.%
   V: 0 - 3.5 Gew.%
   Mo: 0 - 3.5 Gew.%
   Si: 0 - 3.0 Gew.%
   Al: 0 - 3.0 Gew.%
   Cu: 0 - 3.0 Gew.%
   wobei ein kumulierter Gehalt von Co und Mn bei 3.0 - 20.0 Gew.% liegt sowie Eisen und herstellungsbedingte Verunreinigungen den auf 100 Gew.% verbleiben Rest einnehmen und

   (i) eine Wertzahl Cr-eq für Cr-Äquivalente, die sich aus den Gew.%-Anteilen der genannten Legierungskomponenten nach der Formel (1)

$$\text{Cr-eq} = [Cr] + 1.5 \times [Mo] + 0.48 \times [Si] + 2.5 \times [Al] + 1.75 \times [Nb] + 2.3 \times [V] \qquad\qquad (1)$$

   ergibt, größer als 18 ist;
   (ii) eine Wertzahl Ni-eq für Ni-Äquivalente, die sich aus den Gew.%-Anteilen der genannten Legierungskomponenten nach der Formel (2)

$$\text{Ni-eq} = [Ni] + [Co] + 30 \times [C] + 18 \times [N] + 0.1 \times [Mn] - 0.01 \times [Mn]^2 \qquad (2)$$

   ergibt, kleiner als 22 ist;
   (iii) eine Wertzahl PRE für Korrosionsbeständigkeit, die sich aus den Gew.%-Anteilen der genannten Legierungskomponenten nach der Formel (3)

$$PRE = [Cr] + 3.3 \text{ x } [Mo] + 20 \text{ x } [N] \quad (3)$$

ergibt, größer als 25 ist;
(iv) für die Wertzahlen Cr-eq und Ni-eq die Beschränkung nach der Formel (4) gilt

$$Ni\text{-}eq > Cr\text{-}eq - 8 \qquad (4)$$

und
(v) für den Gehalt an Stickstoff und Kohlenstoff folgende Einschränkungen der Formeln (5) und (6) gelten

$$0.25 \leq C + N \leq 1.00 \qquad (5)$$

$$0.25 \leq C/N \leq 1.00 \qquad (6)$$

zur Herstellung eines Stents.

Europäisches
Patentamt

European
Patent Office

Office européen
des brevets

# EUROPÄISCHER RECHERCHENBERICHT

Nummer der Anmeldung

EP 13 16 5600

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2004/062707 A1 (SCIMED LIFE SYSTEMS INC [US]) 29. Juli 2004 (2004-07-29) <br> * Absätze [0001], [0007], [0009], [0010], [0011], [0017], [0025], [0028] - [0036], [0051], [0053] * <br> * Abbildungen 2a-4d * <br> ----- | 1-5 | INV. <br> A61L31/02 <br> C22C38/00 <br> C22C38/18 <br> C22C38/22 <br> C22C38/34 <br> C22C38/38 <br> C22C38/44 <br> C22C38/58 |
| Y | WO 01/00897 A1 (BASF AG [DE]; SPEIDEL MARKUS [CH]) 4. Januar 2001 (2001-01-04) <br> * Seite 1, Zeilen 6-10, 45 - Seite 2, Zeile 1 * <br> * Seite 2, Zeilen 23, 31 * <br> * Seite 4, Zeile 43 - Seite 5, Zeile 14 * <br> * Beispiele 1, 2 * <br> ----- | 1-5 | |
| Y | CH 688 862 A5 (BASF AG [DE]) 30. April 1998 (1998-04-30) <br> * Seite 2, Zeile 3 - Seite 3, Zeile 64 * <br> * Seite 4, Zeilen 10, 11 * <br> * Ansprüche 1, 2 * <br> ----- | 1-5 | |
| Y | EP 0 875 591 A1 (BOEHLER EDELSTAHL [AT] BOEHLER EDELSTAHL GMBH & CO KG [AT]) 4. November 1998 (1998-11-04) <br> * Spalte 2, Zeilen 5-37 * <br> * Spalte 4, Zeile 44 * <br> ----- | 1-5 | RECHERCHIERTE SACHGEBIETE (IPC) <br><br> A61L <br> C22C |

Der vorliegende Recherchenbericht wurde für alle Patentansprüche erstellt

| Recherchenort | Abschlußdatum der Recherche | Prüfer |
|---|---|---|
| Den Haag | 12. September 2013 | Lamers, Wolfram |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.** EP 13 16 5600

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-09-2013

| Im Recherchenbericht angeführtes Patentdokument | Datum der Veröffentlichung | Mitglied(er) der Patentfamilie | Datum der Veröffentlichung |
|---|---|---|---|
| WO 2004062707 A1 | 29-07-2004 | AU 2003303710 A1<br>CA 2512409 A1<br>EP 1581277 A1<br>ES 2416132 T3<br>JP 2006512972 A<br>US 2004129347 A1<br>US 2008069718 A1<br>US 2010114304 A1<br>WO 2004062707 A1 | 10-08-2004<br>29-07-2004<br>05-10-2005<br>30-07-2013<br>20-04-2006<br>08-07-2004<br>20-03-2008<br>06-05-2010<br>29-07-2004 |
| WO 0100897 A1 | 04-01-2001 | AU 5072400 A<br>CN 1373815 A<br>CN 1495281 A<br>EP 1194605 A1<br>JP 3798317 B2<br>JP 2003503595 A<br>US 6682582 B1<br>WO 0100897 A1 | 31-01-2001<br>09-10-2002<br>12-05-2004<br>10-04-2002<br>19-07-2006<br>28-01-2003<br>27-01-2004<br>04-01-2001 |
| CH 688862 A5 | 30-04-1998 | KEINE | |
| EP 0875591 A1 | 04-11-1998 | AT 195767 T<br>DE 59800246 D1<br>DK 875591 T3<br>EP 0875591 A1<br>ES 2150813 T3 | 15-09-2000<br>28-09-2000<br>23-10-2000<br>04-11-1998<br>01-12-2000 |

EPO FORM P0461

Für nähere Einzelheiten zu diesem Anhang : siehe Amtsblatt des Europäischen Patentamts, Nr.12/82